Europäisches Patentamt

European Patent Office

Office européen des brevets

(19)

(11) Publication number: **0 191 214 B1**

# EUROPEAN PATENT SPECIFICATION

(12)

(45) Date of publication of patent specification: **28.08.91**   (51) Int. Cl.⁵: **A61K 7/50**

(21) Application number: **85303502.0**

(22) Date of filing: **17.05.85**

(54) **Purifying liquid cleanser comprising antiseptics.**

(30) Priority: **17.05.84 IL 71865**

(43) Date of publication of application:
**20.08.86 Bulletin 86/34**

(45) Publication of the grant of the patent:
**28.08.91 Bulletin 91/35**

(84) Designated Contracting States:
**CH DE FR GB LI**

(56) References cited:
**CH-A- 83 397**
**GB-A- 5 125**

**CHEMICAL ABSTRACTS, vol. 103, 1985, page
320, abstract no. 11239f, Columbus, Ohio,
US; & IL-A-71 865 (H. RIVKIN) 30-11-1984**

(73) Proprietor: **Rivkin, Haviva
N.B.C. (Natural Biological Cosmetics) Medical House 18, Reines St.
Tel-Aviv(IL)**

(72) Inventor: **Rivkin, Haviva
N.B.C. (Natural Biological Cosmetics) Medical House 18, Reines St.
Tel-Aviv(IL)**

(74) Representative: **Seaborn, George Stephen et al
c/o Edward Evans & Co. Chancery House
53-64 Chancery Lane
London WC2A 1SD(GB)**

Rank Xerox (UK) Business Services

## Description

This invention relates to a cosmetic product or composition for the treatment of blemished, scarred or inflamed skin with acne (comedones).

The first cosmetic products that come into contact with our skin are of the cleansing and protecting kind. The skin of most children during the early years is healthy, soft, free from spots and blemishes and requires little care other than regular cleansing.

At the onset of puberty, however, the skin becomes susceptible to a range of problems, most of which can be attributed to over-activity of the oil-producing sebaceous glands. It has been proved that more than 50% of those effected by excessive spots, pimples, blackheads, acne and related conditions are adolescents between 12 and 18.

The sebaceous glands together with the muscular, nervous and vascular systems are collectively known as the pilosebaceous apparatus and are largely under the control of endogenous hormones which are present in unusually high concentration in the blood during adolescence and puberty. *The corresponding increase in activity of the sebaceous glands themselves gives rise to the production of excessive amounts of sebum. This causes an oiliness of the skin and an increase in the rate of keratinization of the skin's horny layer (the stratum corneum). The horny cells lining the sebaceous follicles also proliferate; they become tightly-packed and can form an occlusive plug or comedone. This physical barrier, coupled with the increased production of sebum, leads to a rapid accumulation of back pressure and the stagnant sabum forms an ideal medium for the proliferation of bacteria (Staphylococcus albus, Staphylococcus aureus and Corynebacterium acnes). When the plugged follicle ruptures and allows the discharge of its contents, local swelling and inflamation are caused. So far no successful topically active substance has yet been found. Acne is usually treated by vitamin A, antibiotics (Tetracycline), various hormonal drugs or subcutaneous hormonal injection.

I have surprisingly discovered that it is possible to prepare a highly effective cosmetic product or composition based on Acidum Boricum, ammonium hydroxide, camphora and hydrogen peroxide.

Acidum Boricum has been used for many years in various substances and compositions of drugs. Acidum Boricum is an antiseptic used in vitro in appropriate dosage.

Ammonium hydroxide is commonly used for skin disinfection.

Camphora is extracted from Laurus, s. Cinnamomum Camphora and is an antiseptic.

Hydrogen peroxide is excellent antibacterial agent, and is used for skin disinfection.

The present invention provides a cosmetic product or composition comprising, as active ingredients per 100 ml of the product or composition:

0.1-6.10 g of boric acid;

0.01-1.6 ml of ammonium hydroxide;

0.02-1.2 g of Camphora; and

0.07-1.1 ml of hydrogen peroxide.

The particular effectivity of the cosmetic product consists in its independent action upon skin, irrespective of the blemishes' origin (hormonal condition, mood, food quality, condition of the digestive system) and independently of drugs, such as antibiotics, vitamins and hormonal drugs.

The product of the invention is effective for local external treatment of problem skin (blemished with scars, acne, comedones),

The product of the invention can be prepared according to conventional cosmetic methods.

The product may comprise the four active components, the balance being the additives commonly used in the cosmetics industry.

Due to its exceptional composition, the cosmetic product of the invention thoroughly cleanses the skin and disinfects the pimpled areas to the maximal extent. The product decreases the secretion of sebum, prevents accumulation of secretion products in the pores and thus prevents the formation of comedones and pimples.

Hereunder are conclusions about the effect upon problem skin of the product of the invention. These conclusions are based upon results received in experimental treatment of a group of patients.

1. Problem skin (blemished with scars, pimples, comedones or blocked with grease) is freed from all these problems as a result of the treatment, the length of the period of treatment needed varying individually and in proportion to the degree of gravity of the situation.

The product entails no addiction; after the treatment is is terminated the patients continue using

* Information in this paragraph is from P. Alexander, S.F. Bloomfield et al., ets: Harry's Cosmeticology. (George Godwin: 1982, 7th edition), Chapter Nine.

other cosmetic products. (These results are valid for patients aged 18-22).

2. The skin is freed from blemishes with no traces left (such as scars or creases) except for cases where the skin is damaged by other treatment or product.

3. Patients with skin blemished with scars or blotches previous to treatment were relieved from these. Creases disappear completely in all cases, whereas the removal of scars or improvement of scarred skin depends on the individual case and is in inverse proportion to the scars' long standing previous to treatment with the product of the invention.

4. Treatment with the product of the invention at an early stage (in ages 12-14) prevents acne completely.

5. The product of the invention has the particular advantage that it is the only agent necessary during the period of treatment - no supplementary dietary or medical treatment need be involved.

The above-mentioned results are true for:

a) a group of patients whose skin problems were never treated before;

b) a group of patients whose case history reveals that conventional medical or cosmetic treatment was of no avail.

Cosmetic products according to the invention are prepared by methods known per se in cosmetic technology.

Examples are given hereunder to illustrate the cosmetic product according to the invention.

Necadas 50 is a trade mark for a product comprising sodium lauryl ether sulfate with 2 mol. EO.

EP 0 191 214 B1

EXAMPLE I

COSMETIC PRODUCT

| Components | Relative proportions |
|---|---|
| Necadas 50 | 35.00 g |
| Boric Acid | 0.10 g |
| Aqua Distillata | 64.00 ml |
| Ammonium Hydroxide | 0.01 ml |
| Camphora | 0.02 g |
| Hydrogen Peroxide | 0.07 g |
| | 100.00 ml |

EXAMPLE II

COSMETIC PRODUCT

| Components | Relative proportions |
|---|---|
| Necadas 50 | 35.00 g |
| Boric Acid | 6.10 g |
| Aqua Distillata | 55.00 ml |
| Camphora | 1.2 g |
| Hydrogen Peroxide | 1.10 ml |
| Ammonium Hydroxide | 1.60 g |
| | 100.00 ml |

Experiments which have been carried out with the products according to the invention have shown that the products are well stabilized and effective after several years' storage under normal temperature conditions.

## Claims

1. A cosmetic product or composition comprising, as active ingredients per 100 ml of the product or composition:
0.1-6.10 g of boric acid;
0.01-1.6 ml of ammonium hydroxide;
0.02-1.2g of Camphora; and
0.07-1.1 ml of hydrogen peroxide.

4

2. A cosmetic product or composition as claimed in claim 1, further comprising additives commonly used in the cosmetics industry.

3. A cosmetic or composition as claimed in claim 2, wherein the product comprises the four components specified in claim 1, the balance being said additives.

4. A cosmetic product or composition as claimed in claim 2 or 3 wherein said additives comprise sodium lauryl ether sulphate and Aqua Distillata.

**Revendications**

1. Produit ou composition cosmétique comprenant, comme ingrédients actifs, par 100 ml du produit ou de la composition :
   0,1-6,10 g d'acide borique ;
   0,01-1,6 ml d'hydroxyde d'ammonium ;
   0,02-1,2 g de camphre ; et
   0,07-1,1 ml de peroxyde d'hydrogène.

2. Produit ou composition cosmétique selon la revendication 1, comprenant, en outre, des additifs utilisés habituellement dans l'industrie des cosmétiques.

3. Produit ou composition cosmétique selon la revendication 2, dans lequel le produit comprend les quatre constituants spécifiés à la revendication 1, le reste étant lesdits additifs.

4. Produit ou composition cosmétique selon la revendication 2 ou 3, dans lequel les additifs sont constitués par le lauryl-sulfate de sodium et de l'eau distillée.

**Patentansprüche**

1. Kosmetisches Produkt oder Zusammensetzung, das als aktive Bestandteile per 100 ml des Produktes bzw. der Zusammensetzung

   0,1 - 6,10 g Bohrsäure,
   0,01 - 1,6 ml Amoniumhydroxyd,
   0,02 - 1,2 g Kampfer, und
   0,07 - 1,1 ml Wasserstoffperoxyd
   enthält.

2. Kosmetisches Produkt oder Zusammensetzung nach Anspruch 1, das weiterhin Additive enthält, wie sie üblicherweise in der kosmetischen Industrie eingesetzt werden.

3. Kosmetisches Produkt oder Zusammensetzung nach Anspruch 2, wobei das Produkt die vier in Anspruch 1 aufgelisteten Komponenten enthält und der Rest auf 100 ml diese Additive ausmachen.

4. Kosmetisches Produkt oder Zusammensetzung nach Anspruch 2 oder Anspruch 3, worin diese Additive Natriumlauryläthersulfat und destilliertes Wasser umfassen.